# EUROPEAN PATENT SPECIFICATION

(11) **EP 1 401 418 B1**
(45) Date of publication and mention of the grant of the patent: **24.12.2008**
(21) Application number: 02739748.8
(22) Date of filing: 06.06.2002
(51) Int. Cl.: A61Q 3/00, A61Q 5/00, A61K 8/44, A61K 8/41

(54) **TOPICAL TREATMENTS USING ALKANOLAMINES**
TOPISCHE BEHANDLUNGEN MIT ALKANOLAMINEN
TRAITEMENTS TOPIQUES UTILISANT DES ALCANOLAMINES

(30) Priority: 06.06.2001 US 875317; 06.07.2001 US 900680; 16.08.2001 US 931616; 27.02.2002 US 85864
(43) Date of publication of application: 31.03.2004
(73) Proprietor: N.V. Perricone LLC, Meriden, CT 06450 (US)
(72) Inventor: Perricone, Nicholas V., Meriden, Connecticut 06450 (US)
(74) Representative: Tanner, James Percival
(86) International application number: PCT/US2002/018026
(87) International publication number: WO 2002/098515

(56) References cited:
- EP-A- 1 192 940
- WO-A-01/01949
- DD-A- 254 326
- DE-A- 3 326 230
- GB-A- 2 220 216
- US-A- 5 554 647
- US-A- 5 945 102
- US-B1- 6 203 793
- PATENT ABSTRACTS OF JAPAN vol. 016, no. 329 (C-0963), 17 July 1992 (1992-07-17) & JP 04 095008 A (KANEBO LTD), 27 March 1992 (1992-03-27)
- DATABASE WPI Section Ch, Week 198710 Derwent Publications Ltd., London, GB; Class D21, AN 1987-071006 XP002374255 & SU 1 243 728 A (MOSC THEATRICAL COSMETIC) 15 July 1986 (1986-07-15)
- DATABASE WPI Week 1979 Derwent Publications Ltd., London, GB; AN 57652B & SU 628 919 A (MOSC SVOBODA COSMETC) 1978
- DATABASE WPI Week 2001 Derwent Publications Ltd., London, GB; AN 435611 & JP 2001 081013 A (SOKEN) 27 March 2001 (2001-03-27)

## Description

### BACKGROUND OF THE INVENTION

Field of the Invention. This invention relates to compositions and methods for improving the appearance and health of, hair, and finger- and toenails, including the promotion of shinier, less brittle hair and nails, the promotion of more elastic hair and nails, providing a more attractive and youthful appearance.

Background of the Invention. Alkanolamines and other acetylcholine precursors and/or ingredients that produce catecholamine activity such as catecholamines and related compounds, alone or in combination with other ingredients and percutaneous penetration enhancers, have been previously described as useful for increasing skin tone and causing subcutaneous muscle contraction when topically applied (U.S. Pat. Nos. 5,554,647 and 5,879,690 to Perricone; these patents and other references, including applications, cited hereafter are expressly incorporated herein in their entireties by reference). Application of these compositions penetrate the skin and shorten subcutaneous muscles, resulting in a lift in tissue on the face, chest, upper arms, upper legs or other areas of application.

The external wrinkled appearance of aging individuals is caused by changes in both epidermal tissue and subcutaneous changes in muscle tissue. In aging, the epidermis thins and skin appendages atrophy. Hair becomes sparse and sebacious secretions decrease, with consequent susceptibility to dryness, chapping, and fissuring. The regularity of tissue structure is lost, and individual cells enlarge, but the total number of cells decreases approximately 30%. Intercellular collagen and elastin increases. The proportion of soluble collagen decreases, and there may be increased cross-linking between long-chain collagen macromolecules. Elastin loses its discrete structure and elasticity, and has an increased calcium content.

Recent studies with the topical application of alkanolamines have led to the observation that the same or similar compositions improve the overall condition of the overlying skin, as well as hair and nails, in a variety of ways, so that topical application of alkanolamines provides an array of desirable effects in addition to subcutaneous muscle contraction.

JP 2001 081013 is directed to hair care products comprising amines to improve hair texture and to improve split and brittle hair. More specifically, JP 2001 081013 discloses a composition comprising amongst other things the alkanolamine, dimethylaminoethanol.

SU 628 919 is directed to a medicinal shampoo composition comprising glutamic acid, citric acid, tartaric acid, vitamin, diethanolamine, disodium ethylene-diamine tetraacetate, glycerol, alcohol, potassium hydroxide and water.

GB 2,220,216 is concerned with a process for washing and conditioning hair comprising applying to the hair a conditioning composition comprising from about 0.05% to about 5% wt. of a surface-active mono or di-cationic amphoteric conditioning agent and from about 0.02% to about 2% wt. of a cationic polymer selected from cationic polysaccharides, homo-polymers of dimethyldiallyl ammonium chloride, co-polymers of dimethyldiallyl ammonium chloride and acrylamide. Further, in one embodiment of a conditioning composition, GB 2,220,216 discloses ethanolamine as a minor ingredient

DE 33 26 230 is concerned with a means for washing or rinsing hair wherein a composition comprising polyaldehyde-carboxylic acids and phosphoric acid salts is contacted with hair. Additionally, DE 33 26 230 discloses a hair cure gel comprising triethanolamine.

DD 254 326 is concerned with the provision of a nail polish remover having improved characteristics comprising amongst other ingredients, triethanolamine.

Compositions of the invention appear to help to reverse membrane damage, including the deleterious cross-linkage and/or cleavage of proteins such as collagen and keratin and lipoprotein and oxidation of membrane lipids and lipoproteins, largely by by stabilizing membranes and stimulating the formation of healthy tissue, result in a variety of desirable effects when applied topically to hair, and nails.

### BRIEF SUMMARY OF THE INVENTION

It is a primary objective of the invention to provide a composition that can be used topically to improve the appearance of hair, and nails, rendering them more uniform and smoother. Hair and nails are rendered more uniform and smoother by softening and adding emolliency to the keratin matrix to increase elasticity and luster.

These and other objectives are accomplished by this invention, which provides topical compositions containing tyrosine and an alkanolamine such as ethylaminoethanol, methylaminoethanol, dimethylaminoethanol, isopropanolamine, triethanolamine, isopropanoldimethylamine, ethylethanolamine, 2-buranolamine, choline, serine, and mixtures thereof, which are applied to mammalian hair and nails to render them more uniform and smoother. Dimethylaminoethanol is particularly preferred. Amounts of active alkanolamine ingredient range from about 0.1 to about 10 % , more narrowly from about 1 % to about 3 %, by weight of the total composition. Adjunct ingredients such as lipoic acid, a fatty acid ester of ascorbic acid, *e.g.,* ascorbyl palmitate, and/or an α-hydroxy acid, *e.g.*, glycolic acid, may be added to formulations of the invention.

Treated hair becomes softer, shinier and more manageable, and nails become less brittle and more lustrous. Pronounced cosmetic benefits are achieved.

### BRIEF DESCRIPTION OF THE INVENTION

In the practice of the invention, compositions that contain tyrosine and an effective amount of an alkanolamine of the formula 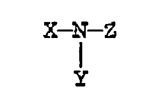 wherein X, Y and Z are selected from the group consisting of hydrogen, C₁-C₃ alkyl groups, C₂₋C₄ alkanol group, wherein at least one of X, Y, or Z is a C₂-C₄ alkanol group bearing at least one hydroxyl group and optionally at least one carboxyl group, are topically applied to hair, or nails to render them more uniform and smoother, As used herein, the term "nails" includes either fingernails or toenails, or both. Useful compounds for the invention include, but are not limited to, ethylaminoethanol, methylaminoethanol, dimethylaminoethanol, isopropanolamine, triethanolamine, isopropanoldimethylamine, ethylethanolamine, 2-butanolamine, choline, serine, and mixtures thereof. Many preferred embodiments employ methylaminoaminoethanol, dimethylaminoethanol, ethylaminoethanol, and/or triethanolamine; particularly preferred is dimethylaminoethanol (DMAE).

The amount of alkanolamine necessary to treat hair, or nails is not fixed *per se,* and necessarily is dependent upon the identity of alkanolamine employed, the amount and type of other active and adjunct ingredients employed, the user's hair, or nail type, and the severity and extent of the conditions treated. Most compositions of the invention contain from about 0.1 % to about 10 % by weight, more narrowly from about 0.25 % to about 5 % to 7 % by weight, and in many cases from about 1% to about 3% by weight, alkanolamine such as dimethylaminoethanol in the total composition, typically in association with a dermatologically acceptable carrier more fully described below. In some examples that follow, efficacious compositions illustrating the invention contain from about 2% to 3% DMAE.

Many alkanolamine compositions of the invention contain at least one adjunct ingredient in addition to active ingredients. Adjunct ingredients include, but are not limited to, lipoic acid, α-hydroxy acids, fatty acid esters of ascorbic acid, and vitamin A and vitamin A derivatives. Many embodiments employ more than one adjunct ingredient. Where employed, adjunct ingredients have additive effects if not synergistic effects due to different mechanism of action.

Alkanolamine compositions of the invention typically are formulated to contain from about 0.01 % to about 6%, more narrowly from about 0.03% to about 5% by weight, and, in many embodiments, from about 0.2% to about 3% by weight tyrosine, based on the total composition. Compositions illustrated in the examples that follow contain from 0.1 to 5 % tyrosine.

As used herein, the term "α-hydroxy acid" has reference to and encompasses the general class of organic compounds containing at least one hydroxy group and at least one carboxyl group, and wherein at least one hydroxyl group is located on the α-carbon atom. Typically, the compounds are organic acids having at least one carboxylic acid group and at least one hydroxyl group on the α-carbon atom, and may contain other functional groups including additional hydroxyl and carboxylic acid moieties. Preferred α-hydroxy acids and/or α-hydroxy acid derivatives are less bulky structurally so that they penetrate the skin well, and thus have a backbone of from one to three carbon atoms such as those more fully described in U.S. Pat. No. 5,965,618 to Perricone at column 6 lines 4 to 29. Where employed, glycolic and/or lactic acid or their derivatives are preferred; glycolic acid is especially efficacious. Glycolic acid or other α-hydroxy acids are typically present in amounts ranging from about 1 % to about 10 % , more narrowly from about 3% to about 7% of the total composition.

Fat-soluble fatty acid esters of ascorbic acid (vitamin C) is employed as an adjunct ingredient in other embodiments, alone or in combination with tyrosine and/or α-hydroxy acids. The more oxidation-resistant saturated fatty acid esters of ascorbic acid are preferred, including, but not limited to, ascorbyl laurate, ascorbyl myristate, ascorbyl palmitate, ascorbyl stearate, and ascorbyl behenate. As is known by skilled workers, ascorbic acid esters include mono-, di-, tri- and tetraesters, and mixtures thereof. Ascorbyl palmitate is used in one embodiment. As denoted herein, where fatty acid esters are described, *e.g*., ascorbyl stearate, compositions having predominantly that ester, *e.g*., predominantly stearate, are included. The esters may be prepared using hydrogenated oils or fats, or fractions thereof, and contain small amounts of another ester. Ascorbyl stearate prepared using canola, for example, commonly contain about 4% ascorbyl palmitate. It is an advantage of the invention that where fatty acid esters of ascorbic acid are employed as an adjunct ingredient, they help stabilize the alkanolamine in the composition. Ascorbyl palmitate and the like ascorbyl esters are typically present in amounts ranging from about 0.5% to about 15%, preferably from about 1% to about 7% to 10%, of the total composition. Vitamin A or vitamin A derivatives may be alternative or additional adjunct ingredients in like concentrations. Vitamin A and vitamin A derivates include, but are not limited to, retinol, retinyl palmitate, retinoic acid, retinal, and retinyl propionate.

Compositions of the invention may be formulated with effective amounts of other active ingredients found in conventional medications or used in therapies with conventional medications.

Lipoic acid is the sulfur ingredient used in some embodiments illustrated hereafter. The term "lipoic acid" encompasses thioctic acid (1,2-dithiolane-3-pen-tanoic acid; 1,2-dithiolane-3-valeric acid), C₈H₁₄O₂S₂, formula weight 206.32, and its reduced form, dihydrolipoic acid. It has been variously known as acetate replacing factor, protogen A, and pyruvate oxidation factor. As used herein, where the properties and advantages of "lipoic acid" (or LA) are discussed as an active ingredient in the practice of the invention, both lipoic acid and its derivatives are encompassed. "Lipoic acid derivatives" include thioctic acid esters, particularly alkyl esters such as fatty acid esters, amides, particularly those isolated from or mimicking naturally occurring lipoamides, salts, particularly alkali metal salts, anhydrides and specifically includes the reduced form, dihydrolipoic acid and its esters, amides and salts. One particularly efficacious derivative that exhibits increased cellular uptake and biological activity useful in the practice of the invention is N,N-dimethyl,N-2-amidoethyl lipoate recently described by Sen, C.K., et al. (Free Radical Biol. Med., 1998, 25: 89) and called lipoic acid plus (LA-Plus). Derivatives may also include those involving other reactive groups known to those skilled in the art. As used herein, the term "derivatives" includes metabolic precursors of lipoic acid. Where lipoic acid derivatives are employed, they must be functionally equivalent to lipoic acid.

Since lipoic acid is both fat- and water-soluble, it is an advantage of the invention that, where employed, lipoic acid can be used as an active ingredient in either lipid or aqueous-based compositions, and it readily crosses cellular membranes and disperses in extracellular and intracellular tissue components. Lipoic acid-containing formulations typically contain from about 0.1% to about 7% by weight lipoic acid. Many embodiments contain more than 1 weight % lipoic acid, *e.g.,* from about 1.1% to about 3 to 5% by weight lipoic acid. One efificacious embodiment contains from about 2 % to about 3% lipoic acid by weight.

Only effective amounts of alkanolamine compositions are needed to provide observable improvement in hair, and nails when used alone, or in combination with other ingredients, so generally topical application is accomplished in association with a carrier, and particularly one in which the alkanolamine active ingredient is soluble *per se* or is effectively solubilized (*e.g*., as an emulsion or microemulsion). Where employed, the carrier is inert in the sense of not bringing about a deactivation or oxidation of the active ingredient(s), and in the sense of not bringing about any adverse effect on the skin areas to which it is applied. In one preferred practice of the invention, the active ingredients are applied in admixture with a dermatologically acceptable carrier or vehicle (*e.g*., as a lotion, cream, ointment, soap, stick or the like) so as to facilitate topical application and, in some cases, provide additional therapeutic effects as might be brought about, *e.g*., by moisturizing of the affected skin and cuticle areas, or the hair. While the carrier for dermatological compositions can consist of a relatively simple solvent or dispersant such as water, it is generally preferred that the carrier comprise a composition more conducive to topical application, and particularly one which will form a film or layer on the skin to which it is applied so as to localize the application and provide some resistance to washing off by immersion in water or by perspiration and/or aid in the percutaneous delivery of the active agent(s). Many preparations are known in the art, and include lotions containing oils and/or alcohols and emollients vegetable oils, hydrocarbon oils and waxes, silicone oils, animal or marine fats or oils, glyceride derivatives, fatty acids or fatty acid esters or alcohols or alcohol ethers, lecithin, lanolin and derivatives, polyhydric alcohols or esters, wax esters, sterols, phospholipids and the like, and generally also emulsifiers (nonionic, cationic or anionic), although some of the emollients inherently possess emulsifying properties. These same general ingredients can be formulated into a cream rather than a lotion, or into gels, or into solid sticks by utilization of different proportions of the ingredients and/or by inclusion of thickening agents such as gums or other forms of hydrophilic colloids. One preferred embodiment is a solution used to saturate a pad used to wipe affected areas; another is a cleanser; another is a shampoo; and others are lotions, creams, and gels. Such compositions are referred to herein as dermally or dermatologically acceptable carriers, and are formulated using conventional techniques known to those of ordinary skill in the art.

Suitable carriers include water, alcohols, oils and the like, chosen for their ability to dissolve or disperse active ingredients. Generally, even low concentrations of active ingredients in a carrier are suitable, depending upon the application regimen and the active and adjunct ingredients employed. Mild hair conditions typically require lower concentrations of active ingredients than do acute conditions. As a practical matter, however, to avoid the need for repeated application, it is desirable that the topically applied composition be formulated to contain the amounts of active ingredients set out above. Generally in the practice of methods of the invention, the composition is topically applied to the affected skin areas as needed to the face, to lesions and scars, often as a tinted cover-up, or at predetermined intervals as a cleanser or a lotion, cream, or gel for the hair, or nails, it generally being the case that gradual improvement is noted with each successive application. Insofar as has been determined based upon clinical studies to date, no adverse side effects are encountered. It is an advantage of the invention that compositions of the invention do not require a pharmaceutical prescription.

Topical compositions of the invention can comprise additional ingredients commonly found in hair care compositions and cosmetics, such as, for example, tinting agents, tinting agents, emollients, skin conditioning agents, emulsifying agents, humectants, preservatives, antioxidants, perfumes, chelating agents, etc., provided that they are physically and chemically compatible with other components of the composition. Preservatives include, but are not limited to, C₁-C₃ alkyl parabens and phenoxyenthanol, typically present in an amount ranging from about 0.5% to about 2.0% by weight percent, based on the total composition. Emollients, typically present in amounts ranging from about 0.01% to 5% of the total composition include, but are not limited to, fatty esters, fatty alcohols, mineral oils, polyether siloxane copolymers, and mixtures thereof. Humectants, typically present in amounts ranging from about 0.1 % to about 5 % by weight of the total composition include, but are not limited to, polyhydric alcohols such as glycerol, polyalkylene glycols (*e.g*., butylene glycol, propylene glycol, dipropylene glycol, polypropylene glycol, and polyethylene glycol) and derivatives thereof, alkylene polyols and their derivatives, sorbitol, hydroxy sorbitol, hexylene glycol, 1,3-dibutylene glycol, 1,2,6-hexanetriol, ethoxylated glycerol, propoxylated glycerol, and mixtures thereof. Emulsifiers, typically present in amounts from about 1% to about 10% by weight of the composition, include, but are not limited to, stearic acid, cetyl alcohol, stearyl alcohol, steareth 2, steareth 20, acrylates/C₁₀₋₃₀ alkyl acrylate crosspolymers, and mixtures thereof. Chelating agents, typically present in amounts ranging from about 0.01 % to about 2% by weight, include, but are not limited to, ethylenediamine tetraacetic acid (EDTA) and derivatives and salts thereof, dihydroxyethyl glycine, tartaric acid, and mixtures thereof. Antioxidants, typically present in an amount ranging from about 0.02 % to about 0.5 % by weight of the composition, include, but are not limited to, butylated hydroxy toluene (BHT); vitamin C and/or vitamin C derivatives, such as fatty acid esters of ascorbic acid, particularly asocorbyl palmitate; butylated hydroanisole (BHA); phenyl-α-naphthylamine; hydroquinone; propyl gallate; nordihydroquiaretic acid; vitamin E and/or derivatives of vitamin E, including tocotrienol and/or tocotrienol derivatives; calcium pantothenates; green tea extracts; mixed polyphenols; and mixtures of any of these. As mentioned above, particularly preferred antioxidants are those that provide additional benefits to the skin such as ascorbyl palmitate. (See additional ingredients and methods in U.S. Pat. Nos. 4,775,530, 5,376,361, 5,409,693, 5,545,398, 5,574,063, 5,643,586, 5,709,868, 5,879,690, 5,965,618, 5,968,618, 6,051,244, 6,162,419, and 6,191,121 to Perricone).

Buffering agents are employed in many compositions. Preferably, the amount of buffering agent is one that results in compositions having a pH ranging from about 4.5 to about 8.5, more preferably from about 5.5 to about 8.5, most preferably from about 6.5 to about 8.0. Typical buffering agents are chemically and physically stable agents commonly found in cosmetics, and can include compounds that are also adjunct ingredients such as citric acid, malic acid, and glycolic acid buffers.

Treatment of hair with compositions of the invention, typically used as a shampoo or rinse, render hair softer, more manageable, and shiny, and the effect is pronounced in the treatment of sun- or chemically bleached hair, including chlorine-bleached hair. Magnification of individual treated hairs show visibly more intact keratin and smoother, less fragmented shanks than untreated controls. Application of alkanolamine compositions to nails results in their becoming more lustrous and considerably less brittle. While not wishing to be bound by any theory, preferred compositions containing lipid penetrants seem to drive active ingredients into the hair shaft and nail surface, softening the keratin matrix, providing emolliency to the keratin and making it more elastic. And, as illustrated in the examples that follow, the effect is cumulative. Successive applications enhance the appearance of both hair and nails.

### EXAMPLES

The following examples are presented to further illustrate and explain the present invention and should not be taken as limiting in any regard. Unless otherwise indicated, all percentages are by weight of the total composition.

### Example 1

Hair rinses formulated to contain 0.1% to 3 % DMAE either in a phospholipid base, a cationic hair conditioner base, or a nonionic shampoo all out-performed the same hair care products without active ingredients in an openended, unblinded comparison of overall hair manageability, softness, and shininess. The effect was more pronounced in subjects with sun- and chlorine-bleached hair.

Fingernails of subjects who soaked the tips of one hand in a solution containing 3% DMAE and 5% tyrosine in a phospholipid base were less brittle and smoother than the fingernails of the other hand soaked in base only. The same effect was seen when the solution was applied topically to the nails or with a brush.

The above description is for the purpose of teaching the person of ordinary skill in the art how to practice the present invention, and it is not intended to detail all those obvious modifications and variations of it which will become apparent to the skilled worker upon reading the description. It is intended, however, that all such obvious modifications and variations be included within the scope of the present invention, which is defined by the following claims. The claims are intended to cover the claimed components and steps in any sequence which is effective to meet the objectives there intended, unless the context specifically indicates the contrary.

## Claims

1. A cosmetic method for treating hair or nails to render them more uniform and smoother comprising applying to the hair or nails, a composition containing tyrosine and an effective amount of an alkanolamine of the formula 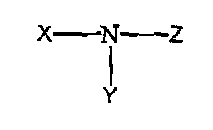 wherein X, Y and Z are selected from the group consisting of hydrogen, C₁-C₃ alkyl groups, C₂-C₄ alkanol groups, wherein at least one of X, Y, or Z is a C₂-C₄ alkanol group bearing at least one hydroxyl group and optionally at least one carboxyl group.

2. A method according to claim 1 wherein the alkanolamine is selected from the group consisting of ethylaminoethanol, methylaminoethanol, dimethylaminoethanol, isopropanolamine, triethanolamine, isopropanoldimethyl- amine, ethylethanolamine 2-butanolamine, choline, serine, and mixtures thereof.

3. A method according to claim 2 wherein the alkanolamine is dimethylaminoethanol.

4. A method according to any of the above claims wherein the alkanolamine is present in the composition in an amount ranging from about 0.1% to about 10% by weight of the composition.

5. A method according to claim 4 wherein the alkanolamine is present in the composition in an amount ranging from about 1 % to about 3 % by weight of the composition.

6. A method according to any of the above claims wherein the composition further comprises at least one adjunct ingredient selected from the group consisting of lipoic acid, an α-hydroxy acid, a fatty acid ester of ascorbic acid, and mixtures of any of these.

7. A method according to claim 6 wherein the α-hydroxy acid is glycolic acid and the fatty acid ester of ascorbic acid is ascorbyl palmitate,

## Patentansprüche

1. Kosmetisches Verfahren zur Behandlung von Haaren oder Nägeln, um sie einheitlicher und glatter zu machen, welches umfaßt, daß man eine Zusammensetzung, welche Tyrosin und eine wirksame Menge eines Alkanolamins der Formel 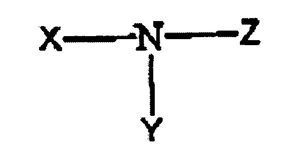 enthält, auf die Haare oder Nägel aufbringt, wobei X, Y und Z aus der Gruppe ausgewählt sind, bestehend aus Wasserstoff, C₁-C₃-Alkylgruppen und C₂-C₄-Alkanolgruppen, wobei wenigstens eine von X, Y oder Z eine C₂-C₄-Alkanolgruppe ist, die wenigstens eine Hydroxylgruppe und optional wenigstens eine Carboxylgruppe trägt.

2. Verfahren nach Anspruch 1, wobei das Alkanolamin aus der Gruppe ausgewählt ist, bestehend aus Ethylaminoethanol, Methylaminoethanol, Dimethylaminoethanol, Isopropanolamin, Triethanolamin, Isopropanoldimethylamin, Ethylethanolamin, 2-Butanolamin, Cholin, Serin und Gemischen davon.

3. Verfahren nach Anspruch 2, wobei das Alkanolamin Dimethylaminoethanol ist.

4. Verfahren nach einem der obigen Ansprüche, wobei das Alkanolamin in der Zusammensetzung in einer Menge im Bereich von etwa 0,1 Gewichts-% bis etwa 10 Gewichts-% der Zusammensetzung vorliegt.

5. Verfahren nach Anspruch 4, wobei das Alkanolamin in der Zusammensetzung in einer Menge im Bereich von etwa 1 Gewichts-% bis etwa 3 Gewichts-% der Zusammensetzung vorliegt.

6. Verfahren nach einem der obigen Ansprüche, wobei die Zusammensetzung weiterhin wenigstens einen zusätzlichen Inhaltsstoff umfaßt, ausgewählt aus der Gruppe, bestehend aus Liponsäure, einer α-Hydroxysäure, einem Fettsäureester von Ascorbinsäure und Gemischen von beliebigen davon.

7. Verfahren nach Anspruch 6, wobei die α-Hydroxysäure Glycolsäure ist und der Fettsäureester von Ascorbinsäure Ascorbylpalmitat ist.

## Revendications

1. Méthode cosmétique pour le traitement des cheveux ou des ongles afin de les rendre plus uniformes et plus lisses, comprenant l'application aux cheveux ou aux ongles d'une composition contenant de la tyrosine et une quantité efficace d'une alcanolamine de formule 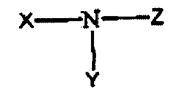 dans laquelle X, Y et Z sont choisis dans le groupe consistant en un atome d'hydrogène, des groupes alkyle en C₁ à C₃, des groupes alcanol en C₂ à C₄, au moins un de X, Y et Z représentant un groupe alcanol en C₂ à C₄ portant au moins un groupe hydroxyle et facultativement au moins un groupe carboxyle.

2. Méthode suivant la revendication 1, dans laquelle l'alcanolamine est choisie dans le groupe consistant en l'éthylaminoéthanol, le méthylaminoéthanol, le diméthylaminoéthanol, l'isopropanolamine, la triéthanolamine, l'isopropanol-diméthylamine, l'éthyléthanolamine, la 2-butanolamine, la choline, la sérine et leurs mélanges.

3. Méthode suivant la revendication 2, dans laquelle l'alcanolamine est le diméthylaminoéthanol.

4. Méthode suivant l'une quelconque des revendications précédentes, dans laquelle l'alcanolamine est présente dans la composition en une quantité allant d'environ 0,1 % à environ 10 % en poids de la composition.

5. Méthode suivant la revendication 4, dans laquelle l'alcanolamine est présente dans la composition en une quantité allant d'environ 1 % à environ 3 % en poids de la composition.

6. Méthode suivant l'une quelconque des revendications précédentes, dans laquelle la composition comprend en outre au moins un ingrédient d'appoint choisi dans le groupe consistant en l'acide lipoïque, un α-hydroxyacide, un ester d'acide gras d'acide ascorbique et des mélanges de n'importe lesquels de ces ingrédients.

7. Méthode suivant la revendication 6, dans laquelle l'α-hydroxyacide est l'acide glycolique et l'ester d'acide gras d'acide ascorbique est le palmitate d'ascorbyle.
